(19) Europäisches Patentamt
European Patent Office
Office européen des brevets

(11) Veröffentlichungsnummer: **0 075 237**
**B1**

(12) **EUROPÄISCHE PATENTSCHRIFT**

(45) Veröffentlichungstag der Patentschrift: **27.11.85**

(51) Int. Cl.⁴: **C 07 C 49/757**, C 07 C 131/10, C 07 C 45/42

(21) Anmeldenummer: **82108423.3**

(22) Anmeldetag: **13.09.82**

(54) **Verfahren zur Herstellung von Derivaten des 1-Formyl-2,6,6-trimethyl-cyclohex-1-ens.**

(30) Priorität: **23.09.81 DE 3137802**

(43) Veröffentlichungstag der Anmeldung: **30.03.83 Patentblatt 83/13**

(45) Bekanntmachung des Hinweises auf die Patenterteilung: **27.11.85 Patentblatt 85/48**

(84) Benannte Vertragsstaaten: **CH DE FR GB LI NL**

(56) Entgegenhaltungen: **US - A - 3 293 301**

(73) Patentinhaber: **BASF Aktiengesellschaft, Carl-Bosch-Strasse 38, D-6700 Ludwigshafen (DE)**

(72) Erfinder: **Janitschke, Lothar, Dr., Wormser Gasse 9a, D-6711 Kleinniedesheim (DE)**
Erfinder: **Hoffmann, Werner, Dr., Ringstrasse 11 c, D-6708 Neuhofen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Bechreibung**

Die vorliegende Erfindung betrifft ein neues Verfahren zur Herstellung von Derivaten des 1-Formyl-2,6,6-trimethyl-cyclohex-1-ens der allgemeinen Formel I

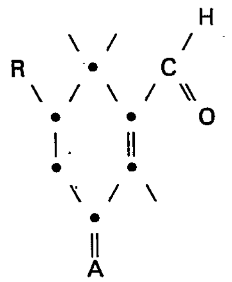 (I)

in der R für -H oder $-CH_3$ steht und A Sauerstoff oder die Oximgruppe bedeutet.

Ausserdem betrifft die Erfindung die Verbindungen I als neue Verbindungen, ausgenommen das 1-Formyl-2,6,6-trimethyl-cyclohex-1-en-3-on (Ia).

Die Verbindung Ia, welche der Verbindung I mit R = -H und A = Sauerstoff entspricht, ist aus der DE-OS 2 724 180 als wertvolles Aromatisierungsmittel für Nahrungsmittel, Getränke, pharmazeutische Präparate und Tabak bekannt.

Wie aus der genannten DE-OS weiter hervorgeht, ist Ia bisher nur auf sehr umständliche Weise erhältlich.

So lässt sich diese Verbindung in einer fünfstufigen Synthese herstellen, indem man 1-Formyl-2,6,6-trimethyl-cyclohex-2-en (IIb) zunächst zur entsprechenden 2,3-Dibromverbindung bromiert, hieraus durch HBr-Abspaltung das entsprechende 3-Brom-cyclohex-2-en gewinnt, das Brom durch den Acetatrest ersetzt, das Acetat hydrolysiert und das entstehende 1-Formyl-2,6,6-trimethyl-cyclohex-1-en-3-ol mit Braunstein zu Ia oxidiert. Auch eine Variante dieses Verfahrens, welche über das 1-Hydroxymethyl-2,6,6-trimethyl-cyclohex-1-en-3-ol verläuft, ist nur wenig befriedigend.

Weiterhin wird in der DE-OS 2 724 180 auf eine vorbekannte Synthese von Ia Bezug genommen, bei der man von der Thioverbindung

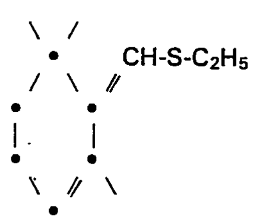

ausgeht, und diese über mehrere Stufen, darunter 2 Bromierungen, in Ia überführt.

Auch die Oxidation von 1-Formyl-2,6,6-trimethyl-cyclohex-1-en (IIa) mit Selendioxid gemäss J. Org. Chem. UdSSR, engl. Ausgabe, Band 4 (1968), S. 308-312 stellt keine brauchbare Methode zur technischen Herstellung von Ia dar, da hierbei die erzielten Ausbeuten nur rund 20% betragen und das Arbeiten mit dem stark giftigen Selendioxid nicht unproblematisch ist.

Der Erfindung lag daher die Aufgabe zugrunde, Ia und darüber hinaus das 5-Methyl-homologe von Ia auf wirtschaftlichere Weise zugänglich zu machen als bisher.

Da ferner Verbindungen mit der Struktureinheit des 1-Formyl-2,6,6-trimethyl-cyclohex-2-ens grosse Bedeutung in der Carotinoid-Chemie und der Chemie der Duft- und Aromastoffe haben, war es weiterhin Aufgabe der Erfindung, die Synthesemöglichkeiten auf diesen Gebieten durch Bereitstellung neuer Zwischenprodukte zu bereichern.

Demgemäss wurde ein neues Verfahren zur Herstellung der eingangs definierten Derivate des 1-Formyl-2,6,6-trimethyl-cyclohex-1-ens gefunden, welches dadurch gekennzeichnet ist, dass man

a) ein 1-Formyl-2,6,6-trimethyl-cyclohex-1-en (IIa) und/oder -cyclohex-2-en (IIb)

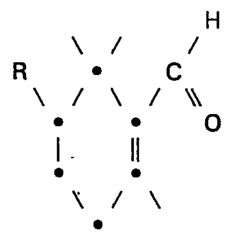

IIa

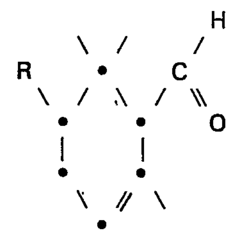

IIb

bei Temperaturen zwischen (—10) und 45°C mit einer Nitrosoverbindung der allgemeinen Formel III

X-NO (III)

umsetzt, welche unter den Reaktionsbedingungen befähigt ist, in ein Anion $X^{\ominus}$ und das Nitrosylkation $[NO]^{\oplus}$ zu spalten, und

b) die hierbei entstehenden Oxime Ib und Ib' (I mit A = NOH)

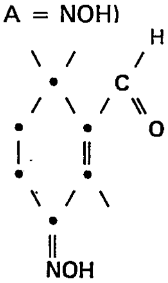

Ib

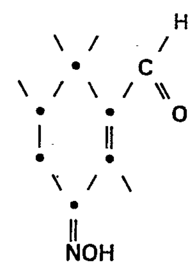

Ib'

zur Herstellung von Ia bzw. Ia' (I mit A = O)

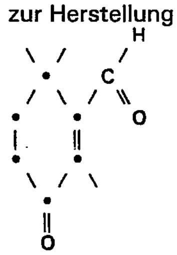

Ia

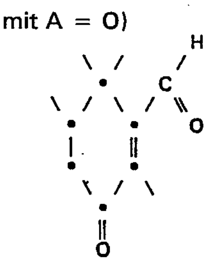

Ia'

in an sich bekannter Weise hydrolysiert.

Als interessante neue Zwischenprodukte werden beansprucht:

1-Formyl-2,6,6-trimethyl-3-oximido-cyclohex-1-en

1-Formyl-2,5,6,6-tetramethyl-3-oximido-cyclohex-1-en

1-Formyl-2,5,6,6-tetramethyl-cyclohex-1-en-3-on.

Die Ausgangsverbindungen IIa und IIb, die man auch in Form eines Gemisches einsetzen kann, sind bekannt und z.B. nach dem in Can. J. Chem. 49 (1971), S. 1764 bis 1766 beschriebenen Verfahren erhältlich.

Das wesentliche Merkmal des erfindungsgemässen Verfahrens ist die Oximierung von IIa bzw. IIb mit der Nitrosoverbindung III, eine Reaktion, die wider Erwarten glatt und einheitlich verläuft und nicht etwa zu einer Addition beider Molekülteile X und NO von III an die Doppelbindung von IIa bzw. IIb führt.

Da es nach den bisherigen Beobachtungen lediglich darauf ankommt, dass das Nitrosierungsmittel III unter den Reaktionsbedingungen ein Nitrosyl-Kation und ein Anion $X^{\ominus}$ auszubilden vermag, ist die sonstige chemische Natur von III von untergeordneter Bedeutung und hat allenfalls Einfluss auf die Reaktionsgeschwindigkeit und die Höhe der Ausbeute. Die Wahl des Nitrosierungsmittels richtet sich aber nicht nur hiernach, sondern auch nach der Gesamtwirtschaftlichkeit des Verfahrens, die jedoch in allen Fällen grösser ist, als bei den bekannten Synthesen.

Geeignete Nitrosierungsmittel, die durch eine Polarität

$$X^{\delta-}\text{-}NO^{\delta+}$$

gekennzeichnet sind, sind beispielsweise Nitrosylhalogenide, wie Nitrosylchlorid, ferner Nitrosylschwefelsäure und Alkylnitrite der Formel IIIa

$$R'\text{-}O\text{-}NO \qquad \text{(IIIa)}$$

in der R' für eine $C_1$- bis $C_8$-Alkylgruppe steht.

Das Nitrosierungsmittel verwendet man für eine vollständige Umsetzung von IIa bzw. IIb in mindestens äquimolaren Mengen. Zweckmässig ist ein Überschuss von bis zu 0,5, vorzugsweise bis zu 0,3 und insbesondere 0,1 Mol pro Mol IIa bzw. IIb. Grössere Überschüsse an Nitrosierungsmittel führen zu verstärkter Bildung von Nebenprodukten.

Die Reaktion erfolgt möglicherweise ausgehend von dem Isomeren IIb. Das Isomere IIa sollte unter den Reaktionsbedingungen im Gleichgewicht mit IIb vorliegen, so dass sich IIb stets nachbilden würde.

In diesem Fall könnte die Reaktion als eine Addition von X-NO an die Doppelbindung in 2,3-Stellung betrachtet werden. Als Zwischenstufe würde dann ein Aldehyd mit einer Nitrosogruppe in 3-Stellung und dem Rest X in 2-Stellung auftreten. Zum Oxim Ib würde man dann beispielsweise durch Abspaltung von $X^{\ominus}$ zur ungesättigten Nitrosoverbindung und durch anschliessende saure Umlagerung der Nitrosoverbindung gelangen.

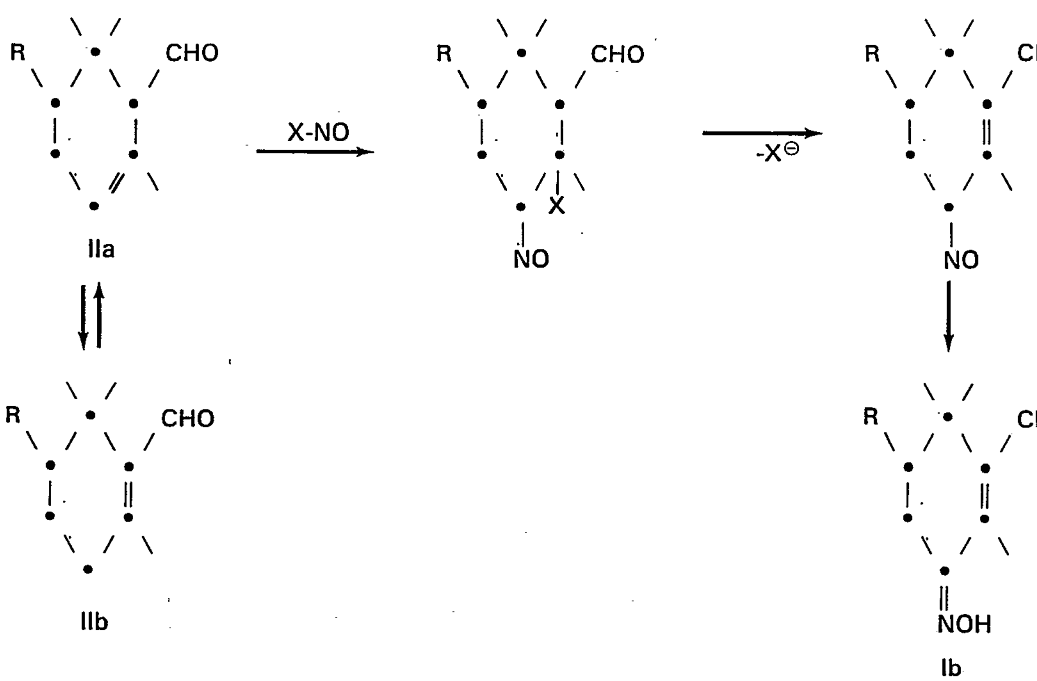


IIa

IIb

Ib

Zur Umlagerung der primär entstehenden NO-Additionsprodukte in die Oxime Ib und Ib' sowie zur Freisetzung der reaktiven Spezies aus Nitrosierungsmitteln wie den Alkylnitriten benötigt man eine Säure.

Daher empfiehlt es sich, die Reaktion in Gegenwart einer starken Mineralsäure, wie Salzsäure oder Schwefelsäure durchzuführen. Bei der Verwendung von Alkylnitriten in aliphatischen Alkoholen arbeitet man zweckmässigerweise mit 2%iger bis gesättigter alkoholischer Salzsäure.

Nitrosylschwefelsäure setzt man zweckmässigerweise als handelsübliche Qualität (ca. 45%ige Nitrosylschwefelsäure in Schwefelsäure) ein. Die Säuren verwendet man im allgemeinen in Mengen von 1,5 bis 35 Mol pro Mol III.

Ferner ist es zweckmäsig, die Umsetzung in homogener Phase in Gegenwart eines Lösungsmittels, wie eines niederen aliphatischen Alkohols oder von flüssigem $SO_2$ vorzunehmen. Mit besonderem Vorteil verwendet man Methanol oder Ethanol. Die Menge des Lösungsmittels beträgt vorzugsweise 2 bis 40 kg pro kg IIa bzw. IIb, insbesondere 2,5 bis 5 kg pro kg IIa bzw. IIb.

Die Reaktionstemperaturen für die Nitrosierung liegen im allgemeinen zwischen (—10) und 45°C, vorzugsweise zwischen 10 und 30°C, insbesondere bei Raumtemperatur.

Zur Durchführung der Reaktion arbeitet man bei diskontinuierlicher Arbeitsweise im allgemeinen so, dass man eine saure Lösung von IIa bzw. IIb vorlegt und hierzu allmählich das Nitrosierungsmittel oder eine Lösung des Nitrosierungsmittels addiert. Bei kontinuierlicher Umsetzung ist es dagegen von Vorteil, die Reaktionspartner bzw. deren Lösungen in konstanten Mengenverhältnissen gleichzeitig durch den Reaktor zu führen.

Die Aufarbeitung des Reaktionsgemisches kann dann wie üblich vorgenommen werden, indem man zunächst die leicht flüchtigen Bestandteile abdestilliert, den Rückstand in einem wasserunlöslichen Lösungsmittel, wie Diethylether aufnimmt und dieses Gemisch mit wässriger Natronlauge versetzt. Das Reaktionsgemisch kann aber nur mit Wasser anstelle von wässriger Natronlauge aufgearbeitet werden, indem man das eingeengte und mit einem wasserunlöslichen Lösungsmittel versetzte Reaktionsgemisch auf eine Mischung von ca. 4,3 kg Eis und ca. 4,3 kg Wasser pro kg eingesetztes IIa bzw. IIb giesst. Die organische Phase wird sodann abgetrennt und vom Lösungsmittel befreit. Das Verfahrensprodukt, welches zunächst meist in Form eines Öles anfällt, kann danach durch Kristallisation, beispielsweise aus Cyclohexan gereinigt werden.

Die weitere meist praktisch quantitative Umsetzung der Oxime Ib bzw. Ib' zu den Ketonen Ia bzw. Ia' erfolgt sodann in üblicher Weise nach den Methoden der Oximspaltung, indem man sie beispielsweise mit wässrig-alkoholischer $NaHSO_3$-Lösung oder mit einer wässrigen Mineralsäure erhitzt. Die Isolierung der Ketone kann dann analog der Isolierung der Oxime oder auch durch Wasserdampfdestillation vorgenommen werden.

Die Oxime Ib und Ib', die man in Ausbeuten von 40 bis über 90% erhält, sind wertvolle Zwischenprodukte für organische Synthesen, beispielsweise für die Herstellung der entsprechenden Ketone Ia und Ia' und einer Reihe bekannter Riech- und Aromastoffe.

Das Keton Ia, das aus Ib in 43 bis 58%iger Reinausbeute gewonnen werden kann, ist ein bekannter Aromastoff und ausserdem ein wichtiges Zwischenprodukt für die Herstellung von Carotinoiden sowie Riech- und Aromastoffen.

Für die neue Verbindung Ia' gilt hinsichtlich ihrer Eignung als Zwischenprodukt das gleiche. Selber ist sie ebenfalls ein wertvoller Riech- und Aromastoff mit einer fruchtigen Duftnote.

*Beispiel 1*

Herstellung von 1-Formyl-2,6,6-trimethyl-3-oximido-cyclohex-1-en (Ib)

Zu einem Gemisch von 1000 ml Methanol und 40 ml konzentrierter Schwefelsäure wird bei 20 bis 25°C 152 g (1 Mol) eines gleichteiligen Gemisches aus 1-Formyl-2,6,6-trimethyl-cyclohex-1-en und -cyclohex-2-en zugegeben, der Ansatz noch 1 Stunde bei Raumtemperatur gerührt, dann bei 20 bis 25°C tropfeneweise mit 323 g (1,2 Mol) einer 43 bis 45%igen schwefelsauren Nitrosylschwefelsäure versetzt und anschliessend noch 1 Stunde bei der Reaktionstemperatur gerührt.

Das Methanol wird bei einer Badtemperatur von 30°C und 26 mbar abdestilliert, wonach der Rückstand in 2000 ml Diethylether aufgenommen und auf eine Mischung aus 3000 g Eis und 480 g 50 gew.-%iger Natronlauge gegossen wird. Die Etherphase wird abgetrennt und die wässrige Phase 4 mal mit je 500 ml Diethylether extrahiert. -

Die übliche Aufarbeitung der vereinigten Etherphasen liefert 176 g Rohoxim (97% der Theorie) in Form eines Öles. Hieraus wird durch Kristallisation aus 1000 ml Cyclohexan das reine Oxim in Form von Kristallen mit einem Schmelzpunkt von 96 bis 100°C in 69%iger Ausbeute gewonnen. Der ölige Rückstand besteht nach dünnschichtchromatographischer Trennung ebenfalls überwiegend aus 1-Formyl-2,6,6-trimethyl-3-oximido-cyclohex-1-en.

Die Identität der Verbindung wurde wie üblich durch verschiedene physikalische Methoden nachgewiesen:

IR-Spektrum (KBr-Pressling):

| | |
|---|---|
| 3260 $cm^{-1}$ | OH |
| 1643 $cm^{-1}$ | C=O |
| 1568 $cm^{-1}$ | C=C |

$^1$H-NMR ($CDCl_3$/220 MHz) δ:

| | |
|---|---|
| 10,33 ppm, s, 1 H, CHO | |
| 10,04 ppm, sbr., 1 H, OH | |
| 2,70 ppm, t (J ~ 7,5 Hz), 2 h, $CH_2$-4 | |
| 2,24 ppm, s, | 3 h, $CH_3$ an C-2 |
| 1,60 ppm, t (J ~ 7,5 Hz), 2 H, $CH_2$-5 | |
| 1,25 ppm s | 6 h, $CH_3$ an C-6 |

$^{13}$C-NMR ($CDCl_3$/20,12 MHz) δ:

| | |
|---|---|
| 193,52 ppm | CHO |
| 157,67 ppm | C-3 |
| 146,24 ppm | C-2 |
| 141,66 ppm | C-1 |
| 36,52 ppm | C-5 |

| | |
|---|---|
| 33,53 ppm | C-6 |
| 26,89 ppm | 2 $CH_3$ an C-6 |
| 19,29 ppm | C-4 |
| 11,81 ppm | $CH_3$ an C-2 |

Elementaranalyse:

| | | | | |
|---|---|---|---|---|
| ber.: | C 66,3 | H 8,3 | O 17,7 | N 7,7 |
| gef.: | C 66,3 | H 8,2 | O 18,0 | N 7,9 |

*Beispiel 2*

Herstellung von Ib

Bei 0 bis 5°C weden 7,6 g (50 mMol) 1-Formyl-2,6,6-trimethyl-cyclohex-1-en zu 100 ml einer gesättigten ethanolischen HCl-Lösung getropft, das erhaltene Gemisch noch 1 Stunde bei 0 bis 5°C gerührt und anschliessend bei 0 bis 5°C tropfenweise mit 5,85 g (60 mMol) n-Pentylnitrit versetzt. Das erhaltene Reaktionsgemisch wird noch 1 Stunde bei 0 bis 5°C gerührt und anschliessend analog Beispiel 1 auf das Oxim aufgearbeitet. Die Ausbeute an reinem kristallinem Ib beträgt 66% d.TH.; weitere Mengen an Ib sind noch in der von der Kristallisation stammenden Mutterlauge enthalten. Sie können hieraus entweder isoliert oder weiter umgesetzt werden.

Ein ähnliches Ergebnis (Reinausbeute 44,2% d. Th.) wird bei Verwendung von 1-Formyl-2,6,6-trimethyl-cyclohex-2-en als Ausgangsverbindung erhalten.

*Beispiel 3*

Bei 0 bis 5°C werden 7,6 g (50 mMol) 1-Formyl-2,6,6-trimethyl-cyclohex-1-en zu einer Mischung von 100 ml Ethanol mit 2 ml konz. Schwefelsäure getropft, das erhaltene Gemisch noch 1 Stunde bei 0 bis 5°C gerührt und anschliessend bei 0 bis 5°C tropfenweise mit 14,7 g einer ca. 43 bis 45%igen schwefelsauren Nitrosylschwefelsäure versetzt. Das erhaltene Reaktionsgemisch wird noch 1 Stunde bei 0 bis 5°C gerührt und anschliessend analog Beispiel 1 auf das Oxim aufgearbeitet. Es resultieren 6,3 g Rohprodukt (69,6% der Theorie). Nach Kristallisation aus 63 ml Cyclohexan erhält man 5,1 g chromatographisch einheitliches Produkt (56,4% d.Th.).

*Beispiel 4*

Herstellung von 1-Formyl-2,5,6,6-tetramethyl-3-oximido-cyclohex-1-en (Ib')

Diese Verbindung wird analog Beispiel 1, ausgehend von einem gleichteiligen Gemisch aus 1-Formyl-2,5,6,6-tetramethyl-cyclohex-1-en und -cyclohex-2-en und 296 g einer ca. 43 bis 45%igen schwefelsauren Nitrosylschwefelsäure in einer Rohausbeute von 72% und einer Ausbeute 36% an reinem kristallinem Produkt vom Fp. = 88-91°C erhalten.

Physikalische Daten der reinen Verbindung:

IR (KBr-Pressling):

| | |
|---|---|
| 3600 - 2700 $cm^{-1}$ | OH |
| 1675 $cm^{-1}$ | C=O |
| 1585 $cm^{-1}$ | C=C |

$^1$H-NMR ($CDCl_3$/80 MHz) δ:

| | |
|---|---|
| 10,29 ppm, s, 1 H, | CHO |
| 2,20 ppm, s, 3 H, | $CH_2$ an C-2 |
| 1,73 ppm, m, 1 H, | CH-5 |
| 1,25 ppm, s, 3 H, | $CH_3$ an C-6 |
| 1,14 ppm, s, 3 H, | |
| 0,98 ppm, d (J I 7 Hz), 3 H, | $CH_3$ an C-5 |

$^{13}$C-NMR ($CDC_3$/90,52 MHz) δ:

| | |
|---|---|
| 194,18 ppm | CHO |
| 157,27 ppm | C-3 |
| 146,52 ppm | C-2 |
| 140,32 ppm | C-1 |
| 37,10 ppm | C-5 |
| 36,81 ppm | C-6 |
| 27,22 ppm | C-4 |
| 25,80 ppm | $CH_3$ an C-6 |
| 20,93 ppm | |
| 15,53 ppm | $CH_3$ an C-5 |
| 11,78 ppm | $CH_3$ an C-2 |

Elementaranalyse:

| | | | | |
|---|---|---|---|---|
| ber.: | C 67,7 | H 8,8 | O 16,4 | N 7,2 |
| gef.: | C 67,9 | H 8,6 | O 16,7 | N 7,2 |

Massenspektrum:

195 m/e $M^{\oplus}$
178 m/e $M^{\oplus}$-17 (-OH)
166 m/e $M^{\oplus}$-29 (-COH)

*Beispiel 5*

Herstellung von 1-Formyl-2,6,6-trimethyl-cyclohex-1-en-3-on (Ia)

45,2 g (0,25 Mol) des Oxims Ib werden zusammen mit 300 ml Ethanol und 300 ml einer 40 gew.%igen wässrigen $NaHSO_3$-Lösung 3 Stunden unter Rückfluss zum Sieden erhitzt. Die übliche Aufarbeitung des Reaktionsgemisches durch Neutralisation und Extraktion mit Ether liefert das Keton Ia in einer Reinausbeute von 57% der Theorie.

Physikalische Daten:

Kp = 54-57°C bei 0,01 mbar;

IR (Film):

| | |
|---|---|
| 1680 $cm^{-1}$ | C=O |
| 1665 $cm^{-1}$ | |
| 1585 $cm^{-1}$ | C=C |

$^1$H-NMR ($CDCl_3$/80 MHz) δ:

| | |
|---|---|
| 10,37 ppm, s, 1 H, | CHO |
| 2,57 ppm, t (J ~ 7 Hz), | 2 H, $CH_2$-4 |
| 2,05 ppm, s, 3 H, | $CH_3$ an C-2 |
| 1,87 ppm, t (J ~ 7 Hz), | 2 H, $CH_2$-5 |
| 1,34 ppm, s, 6 H, | $CH_3$ an C-6 |

$^{13}$C-NMR ($CDCl_3$/90,52 MHz) δ:

| | |
|---|---|
| 200,39 ppm | C-3 |
| 195,65 ppm | CHO |
| 153,83 ppm | C-1 |
| 140,35 ppm | C-2 |
| 37,73 ppm | C-5 |
| 34,38 ppm | C-4 |
| 34,25 ppm | C-6 |
| 26,74 ppm | 2 $CH_3$ an C-6 |
| 10,35 ppm | $CH_3$ an C-2 |

Massenspektrum:
166 m/e M$^{\oplus}$
151 m/e M$^{\oplus}$-15 ($-CH_3$)
137 m/e M$^{\oplus}$-29 (-CHO)

Duftnote: fruchtig, krautig, citronig.

Die Hydrolyse des Oxims mittels verdünnter Schwefelsäure unter gleichzeitiger Entfernung des Ketons durch Wasserdampfdestillation mit anschliessender Fraktionierung liefert das Keton Ia in einer Reinausbaute von 43% der Theorie.

*Beispiel 6*

Herstellung von 1-Formyl-2,5,6,6-tetramethyl-cyclohex-1-en-3-on (Ia')

Die Herstellung erfolgt analog Beispiel 5. Es werden 30 g (0,15 Mol) 1-Formyl-2,5,6,6-tetramethyl-3-oximido-cyclohex-1-en, 190 ml Ethanol und 190 ml einer 40%igen wässrigen $NaHSO_3$-Lösung eingesetzt.

Die Aufarbeitung erfolgt mit 204 ml einer 20%igen $Na_2CO_3$-Lösung. Die Extraktion wurde 5 mal mit je 100 ml Ether durchgeführt.

Rohprodukt: 24,4 g (90,4 d.TH.)

Der reine Aldehyd kann destillativ gewonnen werden.

Kp = 63 bis 65°C bei 0,1 mbar

IR (Film):

| | |
|---|---|
| 1683 $cm^{-1}$ | ungesättigtes Carbonyl |
| 1667 $cm^{-1}$ | ungesättigtes Carbonyl |
| 1600 $cm^{-1}$ | C=C |

$^1$H-NMR /$CDCl_3$/80 MHz) δ:

| | |
|---|---|
| 10,30 ppm, s, 1 H, | CHO |
| 1,99 ppm, s, 3 H, | $CH_2$ an C-2 |
| 1,29 ppm, s, 3 H | $CH_3$ an C-6 |
| 1,23 ppm, s, 3 H | $CH_3$ an C-6 |
| 0,99 ppm, d (J ~ 7 Hz), 3H, | $CH_3$ an C-5 |

$^{13}$C-NMR ($CDCl_3$/90,52 MHz) δ:

| | |
|---|---|
| 200,10 ppm | C-3 |
| 196,19 ppm | CHO |
| 154,70 ppm | C-1 |
| 138,84 ppm | C-2 |
| 42,14 ppm | C-4 |
| 38,92 ppm | C-5 |
| 37,69 ppm | C-6 |
| 20,50 ppm | $CH_3$ an C-6 |
| 20,22 ppm | $CH_3$ an C-6 |
| 15,32 ppm | $CH_3$ an C-5 |
| 10,30 ppm | $CH_3$ an C-2 |

Massenspektrum:
180 m/e M$^{\oplus}$
165 m/e M$^{\oplus}$ -15 ($-CH_3$)
151 m/e M$^{\oplus}$ -29 (-CHO)

Duftnote: fruchtig.

**Patentansprüche**

1. Verfahren zur Herstellung von Derivaten des 1-Formyl-2,6,6-trimethyl-cyclohex-1-ens der allgemeinen Formel I

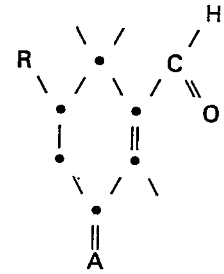

(I)

in der R für -H oder $-CH_3$ steht und A = O oder = NOH bedeutet,
dadurch gekennzeichnet, dass man

a) ein 1-Formyl-2,6,6-trimethyl-cyclohex-1-en (IIa) und/oder -cyclohex-2-en (IIb)

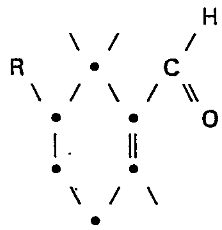

IIa

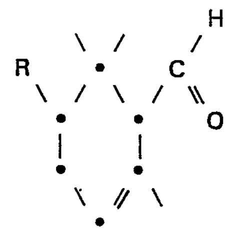

IIb

bei Temperaturen zwischen (-10) und 45°C mit einer Nitrosoverbindung der allgemeinen Formel III

$$X\text{-}NO \qquad (III)$$

umsetzt, welche unter den Reaktionsbedingungen befähigt ist, in ein Anion X$^{\ominus}$ und das Nitrosylkation [NO]$^{\oplus}$ zu spalten, und

b) die hierbei entstehenden Oxime Ib und Ib' (I mit A = NOH)

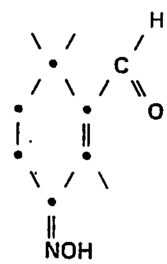

Ib

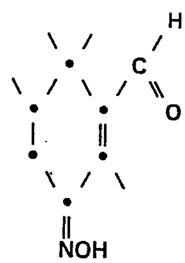

Ib'

zur Herstellung von Ia bzw. Ia' (I mit A = O)

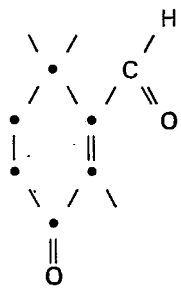

Ia

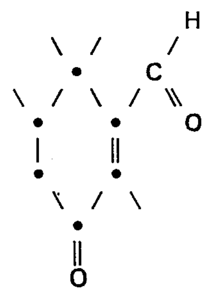

Ia'

in an sich bekannter Weise hydrolysiert.

2. Verfahren nach Anspruch 1, dadurch gekennzeichnet, dass man als Nitrosoverbindung III Nitrosylchlorid, Nitrosylschwefelsäure oder ein $C_1$ bis $C_8$-Alkylnitrit verwendet.

3. Verfahren nach Anspruch 1, dadurch gekennzeichnet, dass man das 1-Formyl-2,6,6-trimethyl-cyclohex-1-en (IIa) und/oder -cyclohex-2-en (IIb) bei Temperaturen zwischen 10 und 30°C mit der Nitrosoverbindung der Formel III umsetzt.

4. Verfahren nach den Ansprüchen 1 und 2, dadurch gekennzeichnet, dass man die Nitrosierung in Gegenwart einer starken Mineralsäure vornimmt.

5. 1-Formyl-2,6,6-trimethyl-3-oximido-cyclohex-1-en.

6. 1-Formyl-2,5,6,6-tetramethyl-3-oximido-cyclohex-1-en.

7. 1-Formyl-2,5,6,6-tetramethyl-cyclohex-1-en-3-on.

**Claims**

1. A process for the preparation of 1-formyl-2,6,6-trimethyl-cyclohex-1-ene derivatives of the general formula I

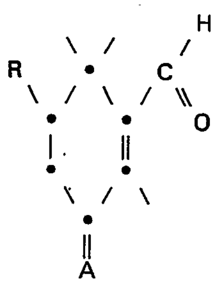

(I)

where R is -H or $-CH_3$ and A is O or NOH, wherein

a) a 1-formyl-2,6,6'-trimethyl-cyclohex-1-ene (IIa) and/or -cyclohex-2-ene (IIb)

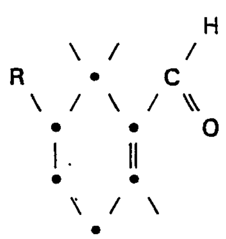

IIa

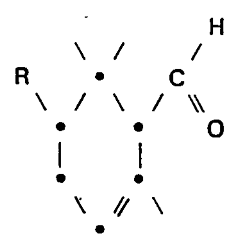

IIb

is reacted, at from —10°C to 45°C, with a nitroso compound of the general formula III

X-NO (III)

which is capable of decomposing under the reaction conditions into an anion $X^{\ominus}$ and the nitrosyl cation $[NO]^{\oplus}$, and

b) the resulting oximes Ib and Ib' (I, where A = NOH)

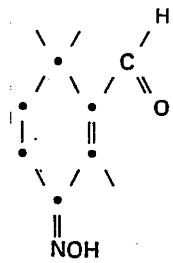

Ib

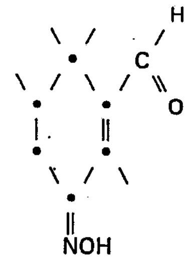

Ib'

are hydrolyzed in a conventional manner to give Ia and Ia' (I, where A = O)

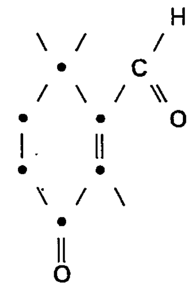

Ia

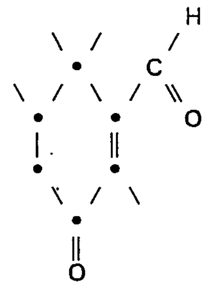

Ia'

2. A process as claimed in claim 1, wherein the nitroso compound III employed is nitrosyl chloride, nitrosyl-sulfuric acid or a $C_1$-$C_8$-alkyl nitrite.

3. A process as claimed in claim 1, wherein 1-formyl-2,6,6-trimethyl-cyclohex-1-ene (IIa) and/or -cyclohex-2-ene (IIb) are reacted with the nitroso compound of the formula III at from 10 to 30°C.

4. A process as claimed in claims 1 and 2, wherein the nitrosylation is effected in the presence of a strong mineral acid.

5. 1-Formyl-2,6,6-trimethyl-3-oximido-cyclohex-1-ene.

6. 1-Formyl-2,5,6,6-tetramethyl-3-oximido-cyclohex-1-ene.

7. 1-Formyl-2,5,6,6-tetramethyl-cyclohex-1-en-3-one.

**Revendications**

1. Procédé pour la préparation de dérivés du 1-formyl-2,6,6-triméthyl-cyclohexène-1 de formule générale

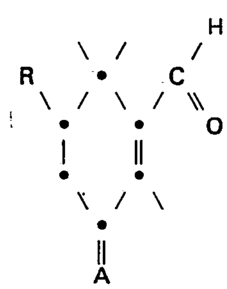

(I)

dans laquelle R est mis pour -H ou $-CH_3$ et A = O ou NOH, caractérisé en ce que:

a) l'on fait réagir, à une temperature comprise entre (—10) et 45°C, un 1-formyl-2,6,6-triméthyl--cyclohexène-1 (IIa) et/ou un 1-formyl-2,6,6-triméthyl-cyclohexène-2 (IIb)

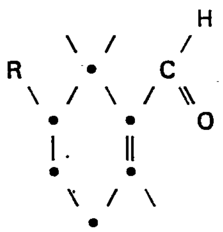

IIa

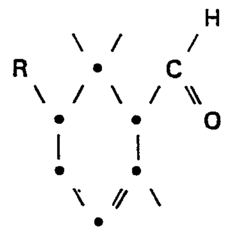

IIb

avec un composé nitroso de formule générale III

X-NO (III)

qui, dans les conditions de la réaction, est susceptible de se scinder en un anion $X^{\ominus}$ et le cation nitrosyle $[NO]^{\oplus}$ et

b) l'on hydrolyse, de façon connue en soi, les oximes Ib et Ib' ainsi obtenues (I avec A = NOH)

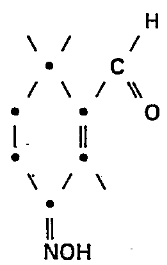

Ib

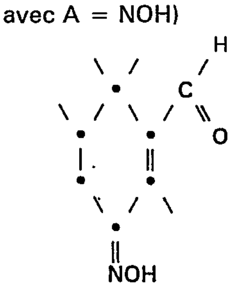

Ib'

pour la préparation de Ia ou Ia' (I avec A = O)

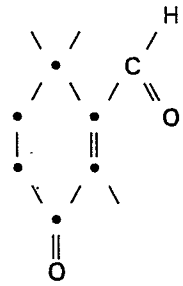

Ia

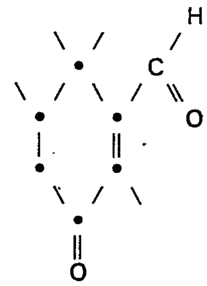

Ia'

2. Procédé selon la revendication 1, caractérisé en ce qu'on utilise, en tant que composé nitroso III, le chlorure de nitrosyle, l'acide nitrosylsulfurique ou un nitrite d'alkyle en $C_1$ à $C_8$.

3. Procédé selon la revendication 1, caractérisé en ce qu'on fait réagir le 1-formyl-2,6,6-triméthyl-cyclohexène-1 (IIa) et/ou le 1-formyl-2,6,6-triméthyl-cyclohexène-2 (IIb) avec le composé nitroso de formule III à des températures comprises entre 10 et 30°C.

4. Procédé selon la revendication 1 ou 2, caractérisé en ce qu'on procède à la nitrosation en présence d'un acide minéral fort.

5. 1-formyl-2,6,6-triméthyl-3-oximido-cyclohexène-1.

6. 1-formyl-2,5,6,6-tétraméthyl-3-oximido-cyclohexène-1.

7. 1-formyl-2,5,6,6-tétraméthyl-cyclohexène-1-one-3.